# EUROPÄISCHE PATENTANMELDUNG

(11) **EP 4 772 598 A1**
(43) Veröffentlichungstag der Anmeldung: **08.07.2026**
(21) Anmeldenummer: 25150203.5
(22) Anmeldetag: 03.01.2025
(51) Int. Cl.: C10M 111/04, A61K 8/00, C10N 70/00, C10N 30/00, C10N 30/20, C10N 20/00, C10N 20/02, C10N 30/02

(54) **MINERALÖLBASIERTES ÖLGEMISCH MIT REDUZIERTEM ÖKOLOGISCHEM FUSSABDRUCK**

(71) Anmelder: Hansen & Rosenthal GmbH & Co. KG, 20457 Hamburg (DE)
(72) Erfinder: HANSEN, Nils, 25451 Quickborn (DE)
(74) Vertreter: Bardehle Pagenberg Partnerschaft mbB Patentanwälte Rechtsanwälte

(57) **Zusammenfassung**

Gegenstand der Erfindung ist ein mineralölbasiertes Ölgemisch mit verbessertem Kohlenstoff-Fußabdruck umfassend 15 - 99,5 Gew.-%mindestens eines mineralischen Öls aus fossilen Quellen und mindestens ein biobasiertes Öl. Das erfindungsgemäße Ölgemisch zeichnet sich durch seinen niedrigen CFP im Vergleich zu Ölen fossilen Ursprungs aus, insbesondere auch im Vergleich zu Re-refined Ölen fossilen Ursprungs. Es bietet zudem die Lagerstabilität herkömmlicher mineralischer Öle fossilen Ursprungs bei gleichzeitig reduziertem ökologischen Fußabdruck. Außerdem können die erfindungsgemäß Ölgemische auf gleiche Weise verarbeitet werden, wie herkömmliche mineralische Öle, so dass anders als bei biobasierten Ölen keine Änderung oder Anpassung der Verarbeitungsprozesse notwendig ist.

## Beschreibung

Gegenstand der Erfindung ist ein mineralölbasiertes Ölgemisch mit verbessertem Kohlenstoff-Fußabdruck bestimmt nach DIN EN ISO 14067.

In der kosmetischen und pharmazeutischen Industrie besteht ein Bedarf an hochreinen Rohstoffen die als Grundlagen, z.B. Salbengrundlagen, Emulsionsgrundlagen oder Lotionsgrundlagen, für kosmetische oder pharmazeutische Zubereitungen verwendet werden können. Bekannte Grundlagen sind beispielsweise Vaseline, halbfestes Paraffinöl, und medizinische Weißöle, wie dickflüssiges Paraffinöl (Paraffinum liquidum) oder dünnflüssiges Paraffinöl (Paraffinum perliquidum). Auf Grund der Verwendung bestehen hohe Anforderungen an die Reinheit der Produkte und deren Hautverträglichkeit, weshalb üblicherweise Kohlenwasserstoffgemische verwendet werden, die frei von aromatischen Kohlenwasserstoffen sind.

Für technische Anwendungen werden auch Paraffingemische eingesetzt, die wegen ihrer an den Anwendungszweck anpassbaren Zusammensetzung hinsichtlich Kettenlänge und Verzweigung für verschiedenste technische Anwendungszwecke verwendet werden können, beispielsweise als Prozessöle für Reifen oder technische Gummiwaren, in Druckfarben oder als technische Spezialöle.

Neben mineralölbasierten Rohstoffen kommen im Zuge eines gestiegenen Umweltbewusstseins zunehmend biobasierte Rohstoffe zum Einsatz. Biobasierte Öle sind umwelttechnisch zu bevorzugen bieten jedoch eine Reihe von technischen Nachteilen. Biobasierte native Produkte, wie z.B. Sonnenblumenöl oder Rapsöl sind häufig nicht ausreichend lagerstabil und können daher in Kosmetika oder Pharmazeutika, wie Salben oder Cremes, nicht herkömmliche Mineralölprodukte ersetzen. Auch erfüllen native biobasierte Öle nicht immer die vorgegeben technischen Parameter für den jeweiligen einsatzzweck.

Viele kommerzielle Produkte basieren bisher auf fossilen Rohstoffen, wie Vaseline oder Weißöl, und die Produktionsprozesse und Verarbeitungsschritte sind auf diese Rohstoffe ausgelegt. Biobasierte Rohstoffe, wie Pflanzenöle, Bienenwachs oder tierische Öle, die theoretisch für die gleichen Anwendung eingesetzt werden könnten, lassen sich nicht auf die gleiche Weise verarbeiten. Ihr Einsatz würde daher eine Umstellung der Rezepturen und der Produktionsprozesse erfordern. Zusätzlich sind viele biobasierte Rohstoffe teuer, so dass bei einem Einsatz von biobasierten Rohstoffen sich der Preis des Endprodukts erhöhen würde.

Eine umweltfreundlichere Variante, die zunehmend zum Einsatz kommt sind aufgearbeitet Mineralöle, die auch als re-refined Öle bezeichnet werden. Diese re-refined Produkte sind gebrauchte Mineralöle, die durch einen speziellen Recyclingprozess wiederaufbereitet werden. Der Aufbereitungsprozess umfasst mehrere Verfahrensschritte, wie z.B. Dehydration und Destillation zur Entfernung der Leichtsieder. Durch das Recyclingverfahren werden Wasser und Verunreinigungen entfernen und die leichtsiedenden Fraktionen abgetrennt und das benutzte Mineralöl wieder nutzbar zu machen.

Re-refined Öle werden häufig in der Automobilindustrie, in Maschinen und in hydraulischen Systemen verwendet. Sie bieten eine umweltfreundliche Alternative zu neuem Mineralöl, da sie helfen, Abfall zu reduzieren und Ressourcen zu schonen, haben jedoch nicht den gleichen Umweltnutzen, wie biobasierte Produkte. Auch können sie in Bereichen mit hohen Reinheitsanforderungen, wie pharmazeutischen Produkten, nur eingeschränkt verwendet werden.

EP 2 071 007 A1 beschreibt eine Zusammensetzung für erneuerbare Basisöle, die aus biologischen Quellen stammen. Die Basisöle basieren auf hydrierten polymethylierten Tripenen und können in verschiedenen Anwendungen eingesetzt werden, einschließlich Motorölen, Hydraulikflüssigkeiten und anderen Schmierstoffen. EP 2 071 007 A1 zielt darauf ab, umweltfreundlichere Alternativen zu herkömmlichen, auf fossilen Brennstoffen basierenden Schmierstoffen zu bieten. Der Ausgangstoff wird von grünen Mikro-Algen erzeugt, weshalb die Herstellung aufwendig und kostenintensiv ist.

Aufgabe der Erfindung ist es ein Ölgemisch bereit zu stellen, das die vorstehenden Nachteile überwindet, das lagerstabil ist, eine hohe Reinheit besitzt und in verschiedenen technischen Anwendungsbereichen von mineralischen Ölen einsetzbar ist, einschließlich kosmetischen und pharmazeutischen Anwendungen.

Die Aufgabe wird erfindungsgemäß gelöst durch ein mineralölbasiertes Ölgemisch mit verbessertem Kohlenstoff-Fußabdruck bestimmt nach DIN EN ISO 14067 gemäß Patentanspruch 1.

Weitere Ausführungsformen sind Gegenstand der Unteransprüche oder nachfolgend beschrieben.

Das erfindungsgemäße mineralölbasiertes Ölgemisch mit verbessertem Kohlenstoff-Fußabdruck bestimmt nach DIN EN ISO 14067 umfasst
- 15 - 99,5 Gew.-% mindestens eines mineralischen Öls aus fossilen Quellen ausgewählt aus
   o Grundölen mit einem Viskositätsbereich von 3 - 500 mm²/s bei 40°C,
   o Schmierstoffgrundölen mit einem Viskositätsbereich von 3 - 4000 mm²/s bei 40°C,
   o Fabrikationsölen mit einem Viskositätsbereich von 3 - 1200 mm²/s bei 40°C,
   o Prozessölen mit einem Viskositätsbereich von 3 - 5000 mm²/s bei 40°C,
   o medizinischen Weißölen (Paraffinum Liquidum gemäß Pharma Eur. /USP/JP/FDA) mit einem Viskositäts-bereich von 3 - 400 mm²/s bei 40°C und
   o technischen Weißölen mit einem Viskositätsbereich von 3 - 500 mm²/s bei 40°C oder
   o Mischungen hiervon, und
- 0,5 - 85 Gew.-% eines biobasierten Isoalkangemischs, wobei das Isoalkangemisch Dimere und Trimere mit einer Kohlenstoffkettenlängen mit 28 bis 54 Kohlenstoffatomen aufweist basierend auf alpha-Olefin-Monomeren mit 14 bis 18 Kohlenstoffatomen, die zumindest teilweise biologischen und nicht fossilen Ursprungs sind und das Isoalkangemisch ggf. hydriert ist und
wobei sich die Komponenten zu 100 Gew.-% ergänzen und die Viskosität bei 40°C nach DIN EN ISO 12185 gemessen ist.

Wenn in der Beschreibung oder den Patentansprüchen die DIN EN ISO 14067 zur Messung des Kohlenstoff-Fußabdrucks genannt ist, so bezieht sich dieses auf die Norm DIN EN ISO 14067 in der Version vom 01.02.2019 herangezogen.

Wenn in der Beschreibung oder den Patentansprüchen die DIN EN ISO 12185 für die Messung der Viskosität bei 40 °C genannt ist, so bezieht sich dieses auf die Norm DIN EN ISO 12185 in der Ausgabe Juni 2024.

Der Kohlenstoff-Fußabdruck, kurz auch CO₂-Fußabdruck, beschreibt die Gesamtmenge an Treibhausgasemissionen, die durch eine Person, ein Produkt, eine Dienstleistung oder ein Unternehmen verursacht werden. Englisch wird der Kohlenstoff-Fußabdruck als Carbon Footprint, abgekürzt CFP, bezeichnet. Diese Emissionen werden üblicherweise in CO₂-Äquivalenten angegeben, um die verschiedenen Treibhausgase vergleichbar zu machen. Eine anerkannte Methode zur Berechnung des Kohlenstoff-Fußabdrucks ist die Norm DIN EN ISO 14067. Diese Norm legt Anforderungen und Richtlinien für die Quantifizierung und Berichterstattung des CO₂-Fußabdrucks von Produkten fest.

Mineralische Öle aus fossilen Quellen werden hauptsächlich durch ihre chemische Zusammensetzung und ihre physikalischen Eigenschaften charakterisiert. Sie bestehen aus verschiedenen Kohlenwasserstoffen, die durch Destillation von Erdöl gewonnen werden. Diese Kohlenwasserstoffe können in drei Hauptgruppen unterteilt werden:
1. Paraffinische Kohlenwasserstoffe: Gesättigte, kettenförmige Kohlenwasserstoffe.
2. Naphthenische Kohlenwasserstoffe: Gesättigte, ringförmige Kohlenwasserstoffe.
3. Aromatische Kohlenwasserstoffe: Ringförmige Kohlenwasserstoffe mit aromatischem Doppelbindungssystem.

Die erfindungsgemäß verwendeten mineralischen Öle weisen bevorzugt einen hohen Anteil paraffinischer Kohlenwasserstoffe von mindestens 40 % der Kohlenwasserstoffe, bevorzugt mehr als 70 %, auf und einen geringen Anteil aromatischer Kohlenwasserstoffe von weniger als 40 % der Kohlenwasserstoffe. Die mineralischen Öle haben bevorzugt einen Gehalt an polyzyklischen Kohlenwasserstoffen (PCA) gemessen nach IP 346 (Ausgabe 01/1992) von <2,99% und einen PAH-Gehalt nach DIN EN 16143 (based on), Ausgabe 05/2013 bestimmt als Summe gemäß Directive 2005/69/EC von kleiner gleich 10 ppm und für Benzo[a]pyrene von kleiner gleich 1 ppm. Ein PAH-Gehalt bestimmt als Summe gemäß Directive 2005/69/EU beinhaltet die Summe der folgenden acht polyzyklischen Aromaten: Benzo(a)pyrene (CAS Nr 50-32-8), Benzo(e)pyrene (CAS Nr. 192-97-2), Benzo(a)anthracene (CAS Nr. 56-55-3), Chrysene (CAS Nr. 218-01-9), Benzo(b)fluoranthene (CAS Nr. 205-99-2), Benzo(j)fluoranthene (CAS Nr. 205-82-3), Benzo(k)fluoranthene (CAS Nr. 207-08-9), Dibenz(a,h)anthracene (CAS Nr. 53-70-3), welche gemäß Bundesrat Drucksache 190/06 vom 09.03.2006, Abschnitt 29 in der Verwendung im Reifen begrenzt sind.

Unter "Schmierstoffgrundöl" wird ein mineralisches Ö verstanden, das aus Rohöl gewonnen und durch verschiedene Raffinationsprozesse veredelt wird. 1 Schmierstoffgrundöl ist der Hauptbestandteil von Schmierstoffen wie Motoren-, Getriebeölen oder Schmierfetten.

"Weißöl", auch als medizinisches Weißöl oder technisches Weißöl bezeichnet, ist ein hochraffiniertes Mineralöl, das nahezu frei von Verunreinigungen ist. Medizinisches Weißöl, auch als Paraffinum Liquidum bezeichnet gemäß Pharma Eur. /USP/JP/FDA, wird häufig in der Kosmetik-, Pharma- und Lebensmittelindustrie verwendet, da es farb- und geruchlos ist und keine toxischen Bestandteile enthält.

Weißöl ist hochraffiniert und nahezu frei von Verunreinigungen, während Schmierstoffgrundöl je nach Raffinationsgrad unterschiedliche Mengen an Verunreinigungen enthalten kann.

Grundöle, die auch Basisöle genannt werden, sind ebenfalls raffinierte Produkte aus Rohöl, bei denen leichte und schwere Kohlenwasserstoffe durch Destillation getrennt werden. Es gibt verschiedene Gruppen von Grundölen, die sich durch ihre Herstellungsmethode und chemischen Eigenschaften unterscheiden. Das American Petroleum Institute (API) legt in seinem Standard fünf Grundöl-Kategorien fest:
- Gruppe I: gering raffinierte Mineralöle mit einem Viskositätsindex von 80-120 gemessen nach ASTM D2270, die weniger als 90 % gesättigte Kohlenwasserstoffe gemessen nach ASTM D2007 enthalten und mehr als 0,03 % Schwefel.

- Gruppe II: Leistungsfähigere Mineralöle, die durch Hydrocracking hergestellt werden, mit einem Viskositätsindex von 80-120 und mehr als oder gleich 90 % gesättigte Bestandteile und ≤ 0,03 Prozent Schwefel.
- Gruppe III: Sehr leistungsfähige Mineralöle, ebenfalls durch starkes Hydrocracking hergestellt, mit einem Viskositätsindex über 120 und ≥ 90 % gesättigte Bestandteile und ≤ 0,03 Prozent Schwefel
- Gruppe IV: Synthetische Öle, die aus Polyalphaolefinen (PAO) bestehen.
- Gruppe V: Umfasst alle anderen Grundöle, die nicht in die ersten vier Gruppen fallen, wie z.B. Ester und Polyester.

Grundöle bzw. Basisöle sind der Hauptbestandteil von Schmierstoffen wie Motorenölen, Getriebeölen und Schmierfetten.

Prozessöle sind raffinierte Mineralölprodukte, die in verschiedenen industriellen Prozessen verwendet werden. Chemisch gesehen bestehen sie aus einer Mischung von Kohlenwasserstoffen, die durch Destillation von Rohöl gewonnen werden, bevorzugt haben diese Öle haben einen Siedebeginn oberhalb von 180°C. Prozessöle werden auch als Fabrikationsöle bezeichnet.

Das biobasierte Isoalkangemisch basiert auf Rohstoffen auf pflanzlicher Basis. Das Isoalkangemisch hat bevorzugt einen Anteil biobasierter Kohlenwasserstoffe von 100% und basiert auf alpha-Olefin-Monomeren mit 14 bis 18 Kohlenstoffatomen (C14-C18-alpha-Olefinen), die biologischen Ursprungs und nicht fossilen Ursprungs sind. Isoalkane sind verzweigte, gesättigte, aliphatische Kohlenwasserstoffe, die früher auch als Isoparaffine bezeichnet wurden. Biobasierte Stoffe bestehen laut EU-Kommission vollständig oder teilweise aus Materialien biologischen Ursprungs, wobei Materialien, die in geologischen Formationen eingebettet oder versteinert ("fossil") sind, ausgeschlossen sind. Biomasse ist laut DIN EN 16575 ein "Material biologischen Ursprungs mit Ausnahme von in geologischen Formationen eingebetteten und / oder zu fossilem Material umgeformten Material". Die ASTM D6866-12 "Standard Test Methods for Determining the Biobased Content of Solid, Liquid, and Gaseous Samples Using Radiocarbon Analysis." ist eine geeignete Methode Materialien zu bewerten, die biobasiert sind und ausgehend von erneuerbaren Rohstoffen hergestellt werden. Eine Messung von 0% ¹⁴C im Verhältnis zum entsprechenden Standard zeigt an, dass der Kohlenstoff vollständig aus fossilen Quellen stammt (z. B. aus Erdöl). Eine Messung von 100% ¹⁴C im Verhältnis zum entsprechenden Standard bedeutet, dass der Kohlenstoff vollständig aus neuen, biogenen Quellen stammt.

Dimerisierung und Trimerisierung dieser Monomere führen zu Isoalkangemischen, die im für die Anwendung interessanten Viskositätsbereich liegen. Die erfindungsgemäßen Isoalkangemische sind daher Di- und Trimere von C₁₄-C₁8 Monomeren und weisen Kohlenstoffkettenlängen von C28 bis C₅₄ auf.

Geeignete Isoalkangemische sind beispielsweise die Produkte SynNova^{®} der Firma Nowi, wie SynNova 4 oder SynNova 9 und die in der Patentanmeldung WO 2016/182930 A1 beschriebenen Isoalkangemische. Das Isoalkangemisch kann auch hydriert sein, so dass hydrierte Isoalkane eingesetzt werden, ebenso kann das Isoalkangemisch teilweise hydriert sein.

Bevorzugt weist das erfindungsgemäße Isoalkangemisch eine kinematische Viskosität von 15 mm²/s bis 80 mm²/s bei 40 °C bestimmt nach DIN EN ISO 3104 (Version 04/2024) auf, besonders bevorzugt 19 bis 75 mm²/s und ganz besonders bevorzugt 20 bis 63 mm²/s.

Das Ölgemisch hat bevorzugt einen Kohlenstofffußabdruck gemessen in g Co₂ eg/kg der mindestens 10 % kleiner ist als der Kohlenstoff-Fußabdruck des reinen mineralischen Öls. Der Kohlenstoff-Fußabdruck ist bevorzugt kleiner als 850 g Co₂ eg/kg gemessen nach DIN EN Iso 14067.

Das mindestens eine mineralische Öl kann ein reines mineralisches Öl oder eine Mischung von 2 bis 10, bevorzugt 2 bis 4, unterschiedlichen mineralischen Ölen sein.

Das mindestens eine mineralische Öl ist bevorzugt ausgewählt aus der Gruppe bestehend aus
- medizinisches Weißöl, auch als Paraffinum Liquidum gemäß Pharma Eur. /USP/JP/FDA bezeichnet, mit einer Viskosität von 10 bis 35 mm²/s, z.B. 17 mm²/s, bei 40°C gemessen nach DIN EN ISO 12185,
- medizinisches Weißöl, Paraffinum Liquidum gemäß Pharma Eur. /USP/JP/FDA, mit einer Viskosität von 38 bis 45 mm²/s, z.B. 40 mm²/s, bei 40°C gemessen nach DIN EN ISO 12185,
- medizinisches Weißöl, Paraffinum Liquidum gemäß Pharma Eur. /USP/JP/FDA, mit einer Viskosität von 50 bis 400 mm²/s, z.B. 70 mm²/s, bei 40°C gemessen nach DIN EN ISO 12185,
- technisches Weißöl mit einer Viskosität von 10 - 120 mm²/s bei 40°C gemessen nach DIN EN ISO 12185,
- Grundöl mit einer Viskosität 10 bis 50 mm²/s, z.B. 30 mm²/s, bei 40°C gemessen nach DIN EN ISO 12185,
- Grundöl mit einer Viskosität 50 bis 500 mm²/s, z.B. 100 mm²/s, bei 40°C gemessen nach DIN EN ISO 12185,
- Schmierstoffgrundöl (SN 150) mit einer Viskosität von 7 bis 80 mm²/s bei 40°C gemessen nach DIN EN ISO 12185,
- Schmierstoffgrundöl (SN 500) mit einer Viskosität von 85 bis 500 mm²/s, z.B. 95 mm²/s, bei 40°C gemessen nach DIN EN ISO 12185,
- Prozessöl mit einer Viskosität von 50 bis 4500 mm²/s bei 40°C gemessen nach DIN EN ISO 12185,
- Basisöl mit einer Viskosität von 10 bis 1200 mm²/s bei 40°C gemessen nach DIN EN ISO 12185
und Mischungen hiervon, bevorzugt Mischungen von 2 bis 4 Ölen hiervon.

Das biobasierte Isoalkangemisch weist bevorzugt einen Anteil biobasierter Kohlenstoffe von 100% (pMC) auf gemessen nach ASTM D6866-22.

Das biobasierte Isoalkangemisch hat bevorzugt einen Viskositätsindex VI gemessen nach DIN ISO 2909, Ausgabe Aug.04, von 120 bis 200, besonders bevorzugt 145-15. Das biobasierte Isoalkangemisch hat bevorzugt folgende Eigenschaften:
- eine Kohlenstoffzahl von C20 bis C70, insbesondere von C32 bis C54;
- ein durchschnittliches Molekulargewicht zwischen 280 und 985 g/mol, insbesondere zwischen 450 und 760 g/mol;
- eine kinematische Viskosität (gemäß ASTM D7042) bei 40 °C zwischen 10 und 80 cSt, insbesondere zwischen 18 und 70 cSt;
- eine kinematische Viskosität (nach ASTM D7042) bei 100 °C zwischen 2 und 20 cSt, insbesondere zwischen 4 und 11 cSt;
- einen Stockpunkt (nach ASTM D5949) von max. -10 °C, insbesondere von max. -18 °C und
- einen Verdampfungsverlust (nach ASTM D5800B) von max. 14 %, insbesondere von max. 10 %.

In einer Ausführungsform besteht das Ölgemisch aus

| | |
|---|---|
| 70 - 99,5 Gew.-% | des mindestens einen mineralischen Öls aus fossilen Quellen und |
| 0,5 - 30 Gew.-% | des biobasierten Isoalkangemisches, |

wobei sich die Komponenten zu 100 Gew.-% ergänzen.

In einer weiteren Ausführungsform besteht das Ölgemisch aus

| | |
|---|---|
| 75 - 99 Gew.-% | des mindestens einen mineralischen Öls aus fossilen Quellen und |
| 1 - 25 Gew.-% | des biobasierten Isoalkangemisches, |

wobei sich die Komponenten zu 100 Gew.-% ergänzen.

In einer weiteren Ausführungsform besteht das Ölgemisch aus

| | |
|---|---|
| 85-99 Gew.% | einer Mischung von 2 bis 4 mineralischen Ölen aus fossilen Quellen wie vorstehend beschrieben und |
| 1 - 15 Gew.-% | des biobasierten Isoalkangemisches, |

wobei sich die Komponenten zu 100 Gew.-% ergänzen.

Das Ölgemisch hat bevorzugt eine Viskosität von 30 bis 40 mm²/s bei 40 °C gemessen nach DIN EN 12185, Version Juni 2024 hat.

Das Ölgemisch hat bevorzugt eine Saybolt-Farbzahl von größer gleich +25 gemessen nach ASTM D 6045 - Version August 2020. Die Farbzahl zeigt an, ob Raffinierie-Produkte, wie z.B. Diesel, Benzin oder Kerosin kontaminiert sind oder durch längere Lagerung beeinträchtigt wurden. Hierfür wird die Saybolt Farbzahl verwendet gemessen nach ASTM D6045 - Version August 2020. Der Saybolt Farbbereich geht von farblos mit einer Sayboltzahl von +30 (klares Wasser / weiß) bis zu einer SayboltZahl von -16 (dunkel gelb).

Bevorzugt weist das erfindungsgemäße Ölgemisch eine Farbzahl-HAZEN von kleiner gleich 20 gemessen nach DIN EN ISO 6271 - 2, Version Mai 2016 auf, besonders bevorzugt <12, ganz besonders bevorzugt <10. Die Farbzahl-Hazen, auch bekannt als APHA-Zahl, ist eine Farbzahl für die Kennzeichnung der Farbe von klaren Flüssigkeiten. Technische Flüssigkeiten haben teilweise durch Verunreinigungen oder Abbauprodukte einen unerwünschten Gelbstich. Die Farbzahl-Hazen wird nach DIN EN ISO 6271-2 (Version Mai 16) durch Vergleich mit Farbstandard-Lösungen in Form von Platin-Cobalt-Farbvergleichslösungen mit Farbzahlen von 0 bis 500 bestimmt. Je kleiner die Farbzahl ist, desto geringer ist die Farbigkeit bzw. der Gelbstich der klaren Flüssigkeit und entsprechend hoch meist die Reinheit der Flüssigkeit.

Das Ölgemisch zeichnet sich durch eine hohe Lagerstabilität aus, die insbesondere gegenüber nativen biobasierten Produkten deutlich erhöht ist und eine Lagerung nicht nur über mehrere Wochen, sondern auch über mehrere Monate erlaubt.

Das erfindungsgemäße Ölgemisch weist gegenüber re-refinded Mineralölen einen deutlich geringeren Kohlenstofffußabdruck, d.h. CFP auf. Es bietet somit die Lagerstabilität herkömmlicher mineralischer Öle fossilen Ursprungs bei gleichzeitig reduziertem ökologischem Fußabdruck. Außerdem können die erfindungsgemäß Ölgemische auf gleiche Weise verarbeitet werden, wie herkömmliche mineralische Öle, so dass anders als bei biobasierten Ölen keine Änderung oder Anpassung der Verarbeitungsprozesse notwendig ist.

Das erfindungsgemäße Ölgemisch zeichnet sich durch seinen niedrigen CFP im Vergleich zu Ölen fossilen Ursprungs aus, insbesondere auch im Vergleich zu Re-refined Ölen fossilen Ursprungs.

Das erfindungsgemäße Ölgemisch weist weiterhin bevorzugt eine Dichte bei 15 °C im Bereich von 0,8 bis 0,9 g/cm³, besonders bevorzugt im Bereich von 0,80 bis 0,85 g/cm³, gemessen nach DIN EN ISO 12185, Version Jun 2024.

Das erfindungsgemäße Ölgemisch hat bevorzugt einen Brechungsindex bei 20 °C im Bereich von 1,4 bis 1,5, besonders bevorzugt von 1,45 bis 1,48, gemessen nach DIN 51423-02, Version Feb 10.

### Beispiele

Es wurden Ölgemische gemäß den in Tabelle 1 und 2 angegebenen Zusammensetzungen hergestellt.

**Tabelle 1: Zusammensetzung der Ölgemische**

| | **Beispiel 1** | **Beispiel 2** | **Beispiel 3** | **Beispiel 4** | **Beispiel 5** | **Beispiel 6** |
|---|---|---|---|---|---|---|
| | **eingesetzt %** | **eingesetzt %** | **eingesetzt %** | **eingesetzt %** | **eingesetzt %** | **eingesetzt %** |
| BIO-basiertes ISO Alkan, hydriert^{a} | 4 | 10 | 1 | 2 | 4 | 10 |
| Med. Weißöl 1^{b} | 80 | 70 | | | | |
| Med. Weißöl 2^{c} | 16 | 20 | 54 | 53 | 51 | 45 |
| Med. Weißöl 3^{d} | | | 45 | 45 | 45 | 45 |

| | | | | | | |
|---|---|---|---|---|---|---|
| a SynNova^{®} 9 H b WOM 40 (CN35_CPS), eigenes Produkt Anmelderin c WOM 16 PF, eigenes Produkt Anmelderin d WOM 71 PF, eigenes Produkt Anmelderin | | | | | | |

**Tabelle 2: Zusammensetzung der Ölgemische**

| | **Beispiel 7** | **Beispiel 8** | **Beispiel 9** | **Beispiel 10** | **Beispiel 11** | **Beispiel 12** | **Beispiel 13** | **Beispiel 14** |
|---|---|---|---|---|---|---|---|---|
| BIO-basiertes Isoalkan^{e}, unhydriert | 1 | 2 | 4 | 10 | 1 | 2 | 4 | 10 |
| Weißöl^{f} | 17 | 16 | 16 | 12 | | | | |
| Grundöl^{g} | 82 | 82 | 80 | 78 | | | | |
| Lubrication Basisöl^{h} | | | | | 85 | 85 | 85 | 85 |
| Lubrication Basisölⁱ | | | | | 14 | 13 | 11 | 5 |

| | | | | | | | | |
|---|---|---|---|---|---|---|---|---|
| e SynNova 9 f WOT 100 PF, eigenes Produkt Anmelderin g KIXX LUBO 150 N (WOT 30), eigenes Produkt Anmelderin h SN 150 (TUD), eigenes Produkt Anmelderin i SN 460 (TUD), eigenes Produkt Anmelderin | | | | | | | | |

Für die Ölgemische wurden die Eigenschaften gemäß den folgenden Methoden gemessen. Die erhaltenen Ölgemische wiesen die in Tabellen 3 und 4 angegebenen Eigenschaften auf.

**Tabelle 3: Eigenschaften gemäß Tabelle 1 hergestellten erfindungsgemäßen Ölgenische**

| | | | | **Beispiel 1** | **Beispiel 2** | **Beispiel 3** | **Beispiel 4** | **Beispiel 5** | **Beispiel 6** |
|---|---|---|---|---|---|---|---|---|---|
| **Analysedaten:** | **Methode** | **Ausgabe:** | **Einheit** | | | | | | |
| Farbe Saybolt | ASTM D 6045 | Aug 20 | ( ) | 30 | 30 | 30 1 | 1 30 1 | 30 | t 30 |
| Farbe Hazen | DIN EN ISO 6271-2 | Mai 16 | ( ) | 10 | 10 | 10 | 10 | 10 | 10 |
| Farbe | ASTM D6045 | Aug 20 | ( ) | | | | | | |
| Dichte 15°C | DIN EN ISO 12185 | Jun 24 | kg/m³ | 861,5 | 859,0 | 858,2 | 857,9 | 857,3 | 855,3 |
| Dichte 20°C | DIN EN ISO 12185 | Jun 24 | kg/m³ | 858 | 856 | 855 | 854,5 | 854 | 852 |
| Viskosität 20°C | DIN ENISO 3104 | Apr 24 | mm²/s | 95,03 | 92,4 | 93,80 | 93,51 | 92,94 | 91,26 |
| Viskosität 40°C | DIN EN ISO 3104 | Apr 24 | mm²/s | 34,9 | 34,5 | 35,2 | 35,2 | 35,1 | 34,80 |
| Viskosität 100°C | DIN EN ISO 3104 | Apr 24 | mm²/s | 5,70 | 5,74 | 5,90 | 5,90 | 5,91 | 5,94 |
| Brechzahl | DIN 51423-02 | Feb 20 | ( ) | 1,4723 | 1,4714 | 1,4711 | 1,4710 | 1,4708 | 1,4700 |
| VDK | DIN 51378 | Dez 20 | ( ) | 0,802 | 0,799 | 0,798 | 0,798 | 0,797 | 0,795 |
| RI | DIN 51378 | Dez 20 | ( ) | 1,043 | 1,0434 | 1,0435 | 1,0436 | 1,0437 | 1,0438 |
| **Aromatische** KW, **X(A)-U** | DIN 51378 U | | % | | | | | | 0 |
| **Naphthen KW,** X(N)-U | | | | 0 | 0 | 0 | 0 | 0 | |
| | | | | 35 | 33 | 32 | 32 | 32 | 30 |
| **Paraff. KW, X(P)-U** | | Dez 20 | | 65 | 67 | 68 | 68 | 68 | 70 |
| Schwefelgehalt | DIN EN ISO 14596 | Dez 07 | % | | | | | | |
| Pour Point | ASTM D 7346 | Jan 15 | °C | -12 | -14 | -12 | -12 | -12 | -13 |
| Flammpunkt COC | DIN EN ISO 2592 | Jan 18 | °C | 213 | 213 | 209 | 209 | 209 | 209 |
| VI | DIN ISO 2909 | Aug 04 | ( ) | 102 | 106 | 110 | 111 | 112 | 115 |
| Verdampfungsverlust1h /250°C | Noack DIN 51581 | Feb 19 | % | 16,5 | 16,9 | 19,6 | 19,8 | 19,6 | 19,4 |
| **PCF*** | **DIN EN ISO 14067** | **01.02.201 9** | **g CO₂eg/k g** | **910** | **835** | **835** | **820** | **800** | **735** |

| | | | | | | | | | |
|---|---|---|---|---|---|---|---|---|---|
| * Der PCF des rein fossilen Vergleichsprodukts liegt bei 935. | | | | | | | | | |

**Tabelle 4; Eigenschaften der gemäß Tabelle 2 hergestellten erfindungsgemäßen Ölgemische**

| | | | | **Beispiel** 7 | Beispiel **8** | **Beispiel** 9 | Beispiel **10** | **Beispiel 11** | **Beispiel 12** | **Beispiel 13** | **Beispiel 14** |
|---|---|---|---|---|---|---|---|---|---|---|---|
| **Analysedaten:** | **Methode** | **Ausgabe:** | **Einheit** | | | | | | | | |
| Farbe Saybolt | ASTM D 6045 | Aug 20 | ( ) | 30 | 30 | 30 | 30 | | | | |
| Farbe Hazen | DIN EN ISO 6271-2 | Mai 16 | ( ) | 10 | 10 | 10 | 10 | | | | |
| Farbe | ASTM D 6045 | Aug 20 | ( ) | | | | | 0,5 h | 0,5 h | 0,5 h | 0,5 h |
| Dichte 15°C | DIN EN ISO 12185 | Jun 24 | kg/m³ | 853,8 | 853,4 | 853,1 | 851,2 | 868,5 | 868,1 | 867,2 | 864,7 |
| Dichte 20°C | DIN EN ISO 12185 | Jun 24 | kg/m³ | 850 | 850 | 850 | 848 | 865 | 865 | 864 | 861,5 |
| Viskosität 20°C | DIN EN ISO 3104 | Apr 24 | mm²/s | 92,53 | 91,86 | 93,28 | 92,01 | 103,29 | 102,76 | 101,70 | 98,62 |
| Viskosität 40°C | DIN EN ISO 3104 | Apr 24 | mm²/s | 35,10 | 34,9 | 35,4 | 35,2 | 37,3 | 37,2 | 37,0 | 36,3 |
| Viskosität 100°C | DIN EN ISO 3104 | Apr 24 | mm²/s | 5,94 | 5,93 | 6,00 | 6,04 | 5,93 | 5,93 | 5,93 | 5,93 |
| Brechzahl | DIN 51423-02 | Feb 20 | ( ) | 1,4696 | 1,4694 | 1,4693 | 1,4686 | 1,4773 | 1,4771 | 1,4768 | 1,4757 |
| VDK | J DIN 51378 | Dez 20 | ( ) | 0,792 | 0,792 | 0,791 | 0,789 | 0,810 | 0,809 | 0,808 | 0,806 |
| RI | DIN 51378 | Dez 20 | ( ) | 1,0442 | 1,0442 | 1,0443 | 1,0445 | 1,0446 | 1,0446 | 1,0447 | 1,0449 |
| Aromatische KW, X(A)-U | DIN 51378 U | Dez 20 | % | 0 | 0 | 0 | 0 | 3 | 3 | 3 | 3 |
| Naphthen KW, X(N)-U | | | | 29 | 29 | 29 | 27 | 31 | 31 | 31 | 29 |
| | | | | 71 | 71 | 71 | 73 | 66 | 66 | 66 | 68 |
| Paraff. KW, X(P)-U | | | | | | | | | | | |

**Tabelle 4: Fortsetzung**

| | | | | Beispiel 7 | Beispiel 8 | Beispiel 9 | Beispiel 10 | Beispiel 11 | Beispiel 12 | Beispiel 13 | Beispiel 14 |
|---|---|---|---|---|---|---|---|---|---|---|---|
| Schwefelgehalt | DIN EN ISO 14596 | Dez 07 | % | 0,0003 | 0,0003 | 0,0003 | 0,0003 | 0,22 | 0,22 | 0,22 | 0,20 |
| Pour Point | ASTM D 7346 | Jan 15 | °C | -25 | -25 | -25 | -26 | -17 | -17 | -18 | -18 |
| Flammpunkt COC | DIN EN ISO 2592 | Jan 18 | °C | 229 | 229 | 230 | 230 | 246 | 246 | 246 | 247 |
| Viskositätsindex | DIN ISO 2909 | Aug 04 | ( ) | 113 | 114 | 115 | 117 | 101 | 101 | 103 | 106 |
| Verdampfungsverlust1 h/250°C | Noack DIN 51581 | Feb. 19 | % | 11,0 | 11,0 | 10,7 | 10,4 | 12,3 | 12,2 | 12,2 | : 12,1 |
| **PCF** | **DIN EN ISO 14067** | **01.02.2019** | **g CO₂eg/kg** | **685** | **670** | **655** | **585** | **615** | **605** | **585** | **520** |

| | | | | | | | | | | | |
|---|---|---|---|---|---|---|---|---|---|---|---|
| *Der PCF des rein fossilen Vergleichsprodukts liegt bei 810. | | | | | | | | | | | |

## Patentansprüche

1. Mineralölbasiertes Ölgemisch mit verbessertem Kohlenstoff-Fußabdruck bestimmt nach DIN EN ISO 14067 umfassend
- 15 - 99,5 Gew.-% mindestens eines mineralischen Öls aus fossilen Quellen ausgewählt aus
∘ Grundölen mit einem Viskositätsbereich von 3 - 500 mm²/s bei 40°C,
∘ Schmierstoffgrundölen mit einem Viskositätsbereich von 3 - 4000 mm²/s bei 40°C,
∘ Fabrikationsölen mit einem Viskositätsbereich von 3 - 1200 mm²/s bei 40°C,
∘ Prozessölen mit einem Viskositätsbereich von 3 - 5000 mm²/s bei 40°C,
∘ medizinischen Weißölen mit einem Viskositätsbereich von 3 - 400 mm²/s bei 40°C und
∘ technischen Weißölen mit einem Viskositätsbereich von 3 - 500 mm²/s bei 40°C oder
∘ Mischungen hiervon, und
- 0,5 - 85 Gew.-% eines biobasierten Isoalkangemischs, wobei das Isoalkangemisch Dimere und Trimere mit einer Kohlenstoffkettenlängen mit 28 bis 54 Kohlenstoffatomen aufweist basierend auf alpha-Olefin-Monomeren mit 14 bis 18 Kohlenstoffatomen, die zumindest teilweise biologischen und nicht fossilen Ursprungs sind und das Isoalkangemisch ggf. hydriert ist und
wobei sich die Komponenten zu 100 Gew.-% ergänzen und die Viskosität bei 40°C nach DIN EN ISO 12185 - Ausgabe Juni 2024 gemessen ist.

2. Ölgemisch gemäß Anspruch 1, **dadurch gekennzeichnet, dass** das Ölgemisch einen Kohlenstofffußabdruck gemessen in g Co₂ eg/kg hat der mindestens 10 % kleiner ist als der Kohlenstoff-Fußabdruck des reinen mineralischen Öls und der Kohlenstoff-Fußabdruck bevorzugt kleiner als 850 g Co₂ eg/kg gemessen nach DIN EN Iso 14067 -Version 01.02.2019 ist.

3. Ölgemisch gemäß Anspruch 1 oder 2, **dadurch gekennzeichnet, dass** das mindestens eine mineralische Öl eine Mischung von 1 bis 5 unterschiedlichen mineralischen Ölen, bevorzugt 2 bis 4 Ölen, ist.

4. Ölgemisch gemäß einem der vorhergehenden Ansprüche, **dadurch gekennzeichnet, dass** das mindestens eine mineralische Öl ausgewählt ist aus der Gruppe bestehend aus
- Medizinisches Weißöl mit einer Viskosität von 10- 35 mm²/s bei 40°C,
- Medizinisches Weißöl mit einer Viskosität von 38 - 45 mm²/s bei 40°C,
- Medizinisches Weißöl mit einer Viskosität von50 - 400 mm²/s bei 40°C,
- Technisches Weißöl mit einer Viskosität von 10 - 120 mm²/s bei 40°C,
- Grundöl mit einer Viskosität 10 - 50 mm²/s bei 40°C,
- Grundöl mit einer Viskosität5 - 500 mm²/s bei 40°C,
- Schmierstoffgrundöl mit einer Viskosität von 7 - 80 mm²/s bei 40°C,
- Schmierstoffgrundöl mit einer Viskosität von 85- 500 mm²/s bei 40°C,
- Prozessöl mit einer Viskosität von 50 - 4500 mm²/s bei 40°C,
- Basisöl mit einer Viskosität von 10 - 1200 mm2/s bei 40°C und Mischungen hiervon.

5. Ölgemisch gemäß einem der vorhergehenden Ansprüche, **dadurch gekennzeichnet, dass** das biobasierte Isoalkangemisch einen Anteil biobasierter Kohlenwasserstoffe von 100% hat.

6. Ölgemisch gemäß einem der vorhergehenden Ansprüche, **dadurch gekennzeichnet, dass** das biobasierte Isoalkangemisch einen Viskositätsindex VI gemessen nach DIN ISO 2909, Ausgabe Aug.04 von 120 bis 200, bevorzugt 145-15, hat.

7. Ölgemisch gemäß einem der vorhergehenden Ansprüche, **dadurch gekennzeichnet, dass** das Ölgemisch besteht aus
70 - 99,5 Gew.-% des mindestens einen mineralischen Öls aus fossilen Quellen und
0,5 - 30 Gew.-% des biobasierten Isoalkangemisches,
wobei sich die Komponenten zu 100 Gew.-% ergänzen.

8. Ölgemisch gemäß einem der vorhergehenden Ansprüche, **dadurch gekennzeichnet, dass** das Ölgemisch eine Viskosität von 30 bis 40 mm²/s bei 40 °C gemessen nach DIN EN 12185, Version Juni 2024 hat.

9. Ölgemisch gemäß einem der vorhergehenden Ansprüche, **dadurch gekennzeichnet, dass** das Ölgemisch eine Saybolt-Farbzahl von kleiner gleich 35 hat gemessen nach ASTM D 6045 - Version August 2020.

10. Ölgemisch gemäß einem der vorhergehenden Ansprüche, **dadurch gekennzeichnet, dass** das Ölgemisch eine hohe Lagerstabilität hat.
